# EUROPEAN PATENT APPLICATION

(11) **EP 1 745 753 A1**
(43) Date of publication of application: **24.01.2007**
(21) Application number: 05022922.8
(22) Date of filing: 20.10.2005
(51) Int. Cl.: A61B 17/54, A45D 29/00

(54) **Foot care device**

(30) Priority: 18.07.2005 US 182953
(71) Applicant: Kotlizky, Amiram, Bakersfield, CA 93312 (US)
(72) Inventor: Kotlizky, Amiram, Bakersfield, CA 93312 (US)
(74) Representative: Becker Kurig Straus

(57) **Abstract**

A device (10) for removing skin from a foot of a user is provided. The device includes a base (12) having a first surface configured for placement against a sole of a foot and an element (14) having an abrasive surface (18) suitable for skin removal. The element is attachable to said base in a manner which enables the user to secure said base against said sole of a first foot and rub a second foot against said abrasive surface.

## Description

### FIELD AND BACKGROUND OF THE INVENTION

The present invention relates to a device configured for foot skin exfoliation, and more particularly to a device which enables hands-free removal of foot skin.

Foot skin build up and hardening (e.g. calluses or corns) is a recurring problem caused by prolonged friction and pressure on the feet. Such skin build up requires periodic treatment for removal of excess skin (skin exfoliation).

Numerous examples of treatment approaches utilizing, for example, medicated pads or skin removal devices (e.g. scrapers, abrasers) are known in the art.

Skin removal devices are generally categorized into hand operated devices and surface-mounted/fixed devices.

Hand operated devices are exemplified by U.S. Pat. Nos. 2,597,525; 3,131,701; 3,733,634; 5,385,532; 5,858,688 and 6,551,262. Such devices are limited in that they require hand operation which can be both tiring and difficult especially when performed in a shower or tub.

Several surface mounted/fixed exfoliation devices configurations have been devised to overcome this limitation of hand operated devices.

One such device is disclosed by U.S. Pat. No. 4,246,914. This device is configured as an elongated bar having an abrasive surface and a smooth surface. In use, the bar is placed on the floor under applied pressure of foot of a user which ensures that the bar remains stationary on the floor. The user then draws his other foot across the abrasive surface in order to remove excess skin.

Although this configuration frees a user from having to use his/her hands, use of such a device can be cumbersome since it requires the user to make sure that the device remains stationary while in use.

There is thus a widely recognized need for, and it would be highly advantageous to have, a device for removing excess skin from feet which would allow a user to stand safely in a shower or tub while using the device.

### SUMMARY OF THE INVENTION

According to one aspect of the present invention there is provided a device for removing skin from a foot of a user comprising a base having a first surface configured for placement against at least a portion of a sole of a foot and an element having an abrasive surface suitable for skin removal, the element being attached to the base in a manner which enables the user to: (i) secure the base against the sole of a first foot; and (ii) rub a second foot against the abrasive surface.

According to further features in preferred embodiments of the invention described below, a second surface of the base is configured as a non-slip surface.

According to still further features in the described preferred embodiments the element is shaped as a band having two ends, whereas the two ends are attachable at opposite sides along a length of the base.

According to still further features in the described preferred embodiments the abrasive surface is 50 to 400 grit sandpaper.

According to still further features in the described preferred embodiments the device enables the user to rub the second foot against the abrasive surface while the first foot is stationary against a floor surface.

According to still further features in the described preferred embodiments the band functions as a strap.

According to still further features in the described preferred embodiments an angle of attachment of the two ends of the strap is selected so as to form a variable width strap.

According to still further features in the described preferred embodiments the base is configured with two slits each being for accepting an end of the two ends of the band.

According to still further features in the described preferred embodiments a dimension and shape of the base is selected such that when secured against the at least a portion of the sole, the surface of the base contact at least a portion of the sole.

According to another aspect of the present invention there is provided a kit for removing skin from a foot of a user comprising at least one base having a first surface configured for placement against a portion of a sole of a foot and a plurality of elements each having an abrasive surface of a specific abrasive quality each being suitable for skin removal, the at least one base being configured capable of attachment to an element of the plurality of elements.

According to still further features in the described preferred embodiments the attachment forms a device which enables the user to: (i) secure a base of the at least one base against the sole of a first foot; and (ii) rub a second foot against the abrasive surface.

According to still further features in the described preferred embodiments a second surface of the base is configured as a non-slip surface.

According to still further features in the described preferred embodiments the plurality of elements is shaped as a band having two ends, whereas the two ends are attachable at opposite sides along a length of the at least one base.

According to still further features in the described preferred embodiments the abrasive surface is 50 to 400 grit sandpaper.

According to still further features in the described preferred embodiments the band functions as a strap.

According to still further features in the described preferred embodiments an angle of attachment of the two ends of the strap to the at least one base is selected so as to form a variable width strap.

According to still further features in the described preferred embodiments the at least one base is configured with at least two slits each being for accepting an end of the two ends of the band.

According to still further features in the described preferred embodiments a dimension and shape of the at least one base is selected such that when secured against the at least a portion of the sole, the surface of the at least one base contact at least a portion of the sole.

The present invention successfully addresses the shortcomings of the presently known configurations by providing a hands free foot skin removal device which is easy to use.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIGs. 1-5 illustrates various views of one preferred embodiment of the device for foot skin removal of the present invention.
FIG. 6 is a perspective view of the abrasive surface element of the device of the present invention shown in Figures 1-5.

### DESCRIPTION OF THE PREFERRED EMBODIMENTS

The present invention is of a device and kit which can be used for removal of foot skin. Specifically, the present invention can be used to remove dead skin from a user's foot without need for hand operation.

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

The prior art is replete with devices and systems which can be used for removal of foot skin. However, the art known devices either require a user to manually operate the device or are cumbersome to use.

The present device overcomes the limitations of prior art devices by providing a device and kit which can be used hands free in the shower or other confined spaces, while standing up and without need for hand operation.

Thus, according to one aspect of the present invention there is provided a device for removing skin from a foot of a user. The device is constructed from a base which has a first surface configured for placement against at least a portion of a sole of a foot (e.g., a front portion) and an element which includes an abrasive surface suitable for skin removal. Attachment between the base and element is effected in a manner which enables the user to secure the base against sole of a first foot and rub a second foot against the abrasive surface of the element.

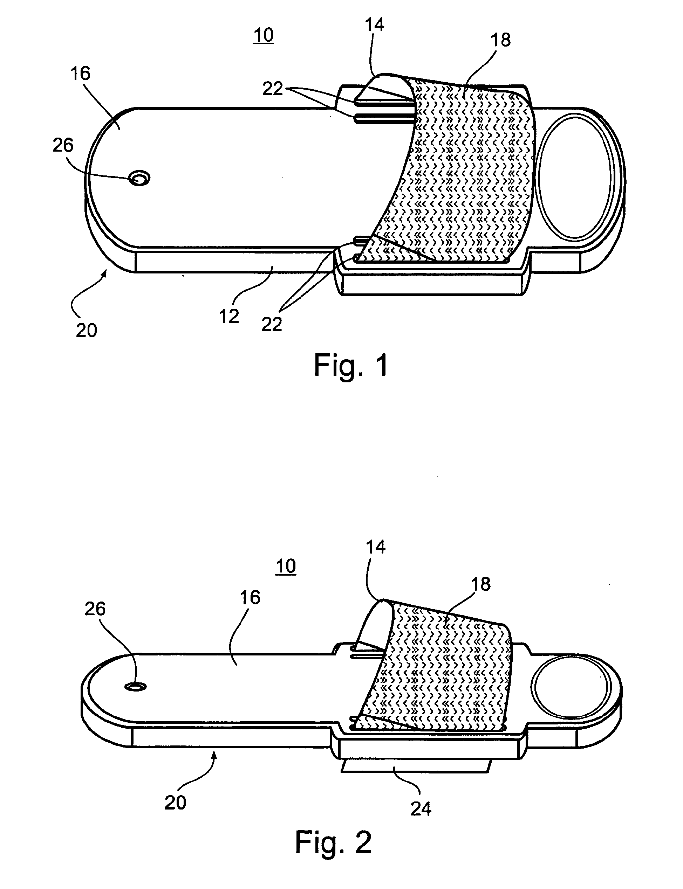

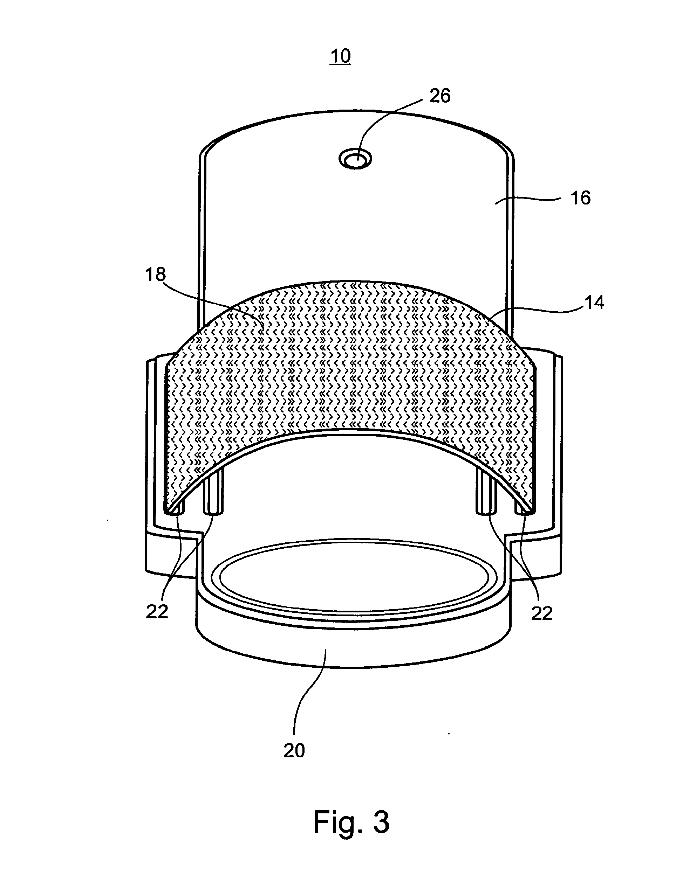

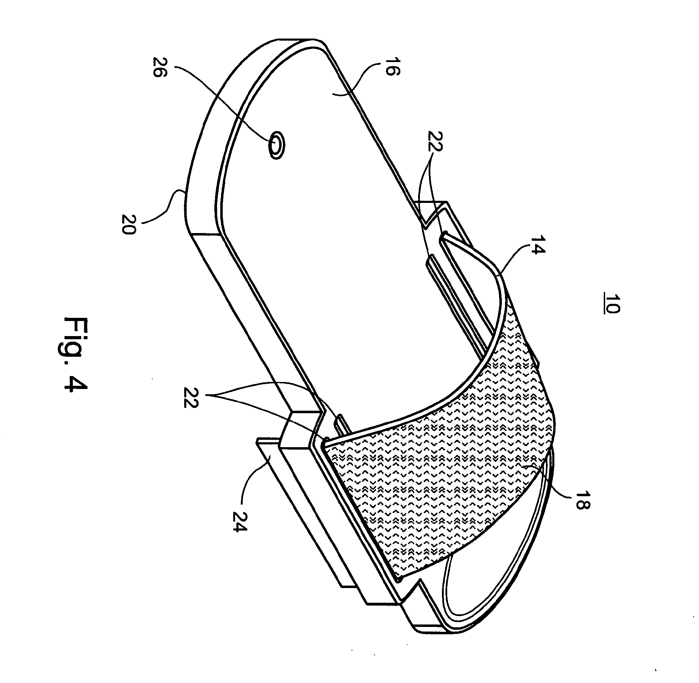

Referring now to the drawings, Figures 1-6 illustrates the device of the present invention which is referred to hereinunder as device 10.

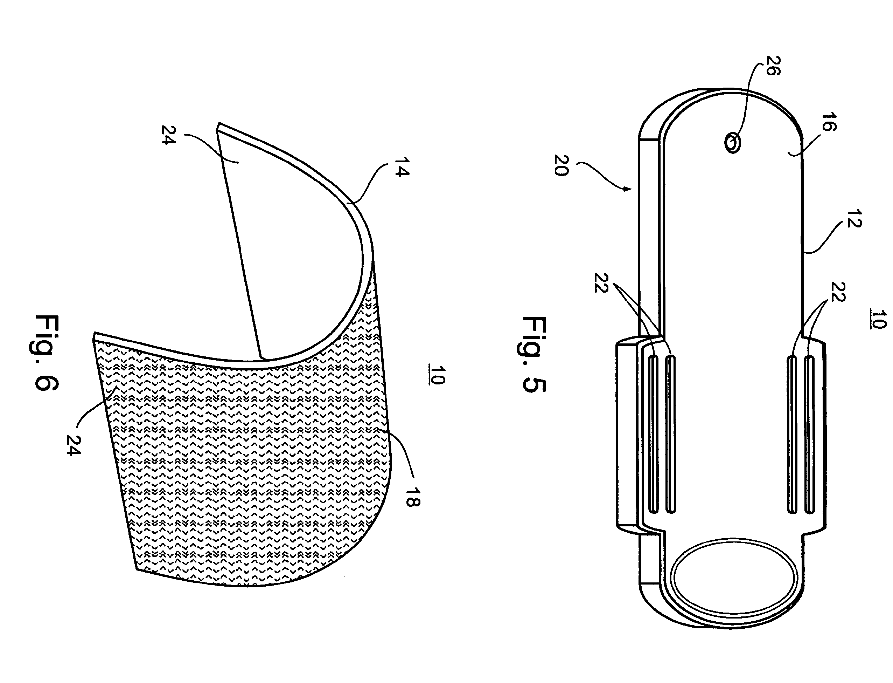

Device 10 includes a base 12 (shown separately in Figure 5) and an element 14 (shown separately in Figure 6) which is attachable to base 12. Base 12 is adapted for placement against a sole of a user and as such includes a first surface 16 which is configured for such use. It will be appreciated that although Figures 1-5 illustrate a sole-like base 12, that a base which can be fitted against any portion of a sole (e.g., front portion) is envisaged. Base 12 can be fabricated from any material and at any size depending on the foot of the user and sole coverage desired. Suitable materials include wood, metals, carbon fiber and polymers such as nylon, plastics and the like, such materials can be cast, extruded or injected molded to form the shape of base 12. Since foot skin exfoliation is frequently performed in the shower, base 12 is preferably fabricated from water resistant material and includes a second and non-slip surface 20 (e.g., configured with ridges or coated with neoprene).

Base 12 is preferably fabricated in dimension suitable fro placement against a the entire foot sole of a user or any desired portion thereof. As such, a typical length ranges between 5-40 cm, while a width is selected from a range of 7-15 cm. It should be noted that a variable width base which follows the contour of a user's sole (similar in shape to an insole) is also envisaged. The width of base 12 is selected so as to endow it with sufficient rigidity when in use, typically 0.5-1.5 cm depending on the material utilized for fabrication.

Element 14 includes at least one abrasive surface 18 which is selected capable of removing skin when rubbed thereagainst. Element 14 can configured in any shape so long as its attachable to base 12 and enables securing the foot of a user to base 12. A preferred shape is a strip or band (Figure 6) of a length selected from a range of 8-30 cm and a width selected from a range of 2-10 cm. Various types of abrasive surfaces 18 can be formed on, or attached to, element 14. Examples include surfaces configured with groves, hooks, teeth and the like. Various abrasive coating which can be used in element 14 are available from the #M corporation. A preferred abrasive surface 18 is that formed from sandpaper. Any type of sandpaper or sandpaper like surfaces can be used in element 14, a grit of 40-500 is preferred. Examples of sandpaper surfaces that can be utilized by the present invention, include, but are not limited to any dry or wet sandpaper, such as that commercially available from the 3M corporation (cms.3m.com/cms/US/en/2-188/kriilFR/view.jhtml).

One advantage to a sandpaper abrasive surface 18, is that element 14 can be entirely fabricated from a sandpaper strip or band, thereby reducing cost of fabrication.

As is mentioned hereinabove, element 14 is attachable to base 12 in a manner which enables securing first surface 16 to a sole of a first foot of the user while enabling skin removal from a second foot without manual operation.

Such attachment can be effected via any one of several approaches including gluing, sewing, welding, pinning or stapling.

Preferably, element 14 is attached to base 12 via slits 22 (four shown in Figure 5) which are formed along opposite sides of base 12. Such slits are configured such that threading of ends 24 of element 14 therethrough, attaches element 14 to base 12 to form a loop which resembles a strap of sandal. Use of slits 22 for attachment enable a user to set a preferred loop size (via slit selection and/or length of element ends 24 threaded therethrough) while it also enables easy replacement of element 14 when necessary (e.g., when abrasive surface 18 is worn out). Use of slits 22 is also advantageous in that it enables securing of element 14 to base 12 by simply folding over ends 24 of element 14 against surface 20. Subsequent stepping on base 12 ensures that element 14 remains secured in place.

Device 10 can be packaged as apart of a kit which includes one or more base sizes and several element 14 of several sizes and/or abrasive surface grades/grits.

In use, a user simply connects element 14 to base 12 and secures assembled device 10 against a sole of one foot (left or right). The user then stands the foot on base 12 of device 10 and rubs (while standing or sitting) the other foot against abrasive surface 18 of element 14 until sufficient skin removal is achieved. When skin removal is complete, device 10 is removed from the foot and stored, e.g. hung on a shower wall from hole 26.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention.

## Claims

1. A device for removing skin from a foot of a user comprising a base having a first surface configured for placement against at least a portion of a sole of a foot and an element having an abrasive surface suitable for skin removal, said element being attachable to said base in a manner which enables the user to:
(i) secure said base against said at least a portion of said sole of a first foot; and
(ii) rub a second foot against said abrasive surface.

2. The device of claim 1,
wherein a second surface of said base is configured as a non-slip surface; or
wherein said abrasive surface is 50 to 400 grade sandpaper; or
wherein the device enables the user to rub said second foot against said abrasive surface while said first foot is stationary against a floor surface; or
wherein a dimension and shape of said base is selected such that when secured against said at least a portion of said sole, said surface of said base contact at least a portion of said sole.

3. The device of claim 1, wherein said element is shaped as a band having two ends, whereas said two ends are attachable at opposite sides along a length of said base.

4. The device of claim 3,
wherein said band functions as a strap, preferably wherein an angle of attachment of said two ends of said strap is selected so as to form a variable width strap; or
wherein said base is configured with two slits each being for accepting an end of said two ends of said band.

5. A kit for removing skin from a foot of a user comprising at least one base having a first surface configured for placement against a portion of a sole of a foot and a plurality of elements each having an abrasive surface of a specific abrasive quality each being suitable for skin removal, said at least one base being configured capable of attachment to an element of said plurality of elements.

6. The kit of claim 5,
wherein said attachment forms a device which enables the user to:
(i) secure a base of said at least one base against said at least a portion of said sole of a first foot; and
(ii) rub a second foot against said abrasive surface;
preferably wherein a dimension and shape of said at least one base is selected such that when secured against said at least a portion of said sole, said surface of said at least one base contact at least a portion of said sole.

7. The kit of claim 5,
wherein a second surface of said base is configured as a non-slip surface; or
wherein said abrasive surface is 50 to 400 grade sandpaper.

8. The kit of claim 5, wherein each of said plurality of elements is shaped as a band having two ends, whereas said two ends are attachable at opposite sides along a length of said at least one base.

9. The kit of claim 8,
wherein said band functions as a strap;
preferably wherein an angle of attachment of said two ends of said strap to said at least one base is selected so as to form a variable width strap.

10. The kit of claim 8, wherein said at least one base is configured with at least two slits each being for accepting an end of said two ends of said band.
